# EUROPEAN PATENT SPECIFICATION

(11) **EP 3 603 592 B1**
(45) Date of publication and mention of the grant of the patent: **16.11.2022**
(21) Application number: 18775724.0
(22) Date of filing: 16.03.2018
(51) Int. Cl.: A61F 13/537, A61F 13/53, A61F 13/534, A61F 13/472

(54) **ABSORBENT ARTICLE**
ABSORBIERENDER ARTIKEL
ARTICLE ABSORBANT

(30) Priority: 30.03.2017 JP 2017067158
(43) Date of publication of application: 05.02.2020
(73) Proprietor: Daio Paper Corporation, Ehime 799-0492 (JP)
(72) Inventor: TAGOMORI, Junta, Sakura-shi Tochigi 329-1411 (JP)
(74) Representative: Epping - Hermann - Fischer
(86) International application number: PCT/JP2018/010503
(87) International publication number: WO 2018/180608

(56) References cited:
- EP-A1- 2 486 901
- EP-A1- 2 907 493
- WO-A1-2007/035038
- WO-A1-2011/043256
- WO-A1-2013/099634
- JP-A- 2008 284 190
- JP-A- 2010 088 528
- JP-A- 2015 066 382
- US-A- 5 281 207
- US-A1- 2012 136 329

## Description

### TECHNICAL FIELD

The present invention mainly relates to absorbent articles used for, for example, an incontinence pad, a sanitary napkin, a pantiliner, a medical pad, toiletries, and a disposable diaper.

### BACKGROUND ART

A known absorbent article includes an impermeable back-side sheet such as a polyethylene sheet or a polyethylene-sheet-laminated nonwoven fabric, a permeable upper-side sheet such as a nonwoven fabric or a permeable plastic sheet, and a polymer sheet, so-called a pulp-less absorber, that includes no pulp fiber and is disposed between the impermeable back-side sheet and the permeable upper-side sheet.

An absorbent article using a polymer sheet has advantages that the thickness of an absorber can be reduced, and the wearing discomfort can be reduced.

However, it has been pointed out that a polymer sheet has the following disadvantages: (1) because the water absorption rate of superabsorbent polymers is generally slower than that of absorbent fibers such as pulp, the polymer sheet cannot quickly absorb a body fluid discharged quickly; (2) gel blocking, where superabsorbent polymer particles absorb water, swell, adhere tightly to each other, and prevent passage of a body fluid, tends to occur; and (3) when a large amount of a body fluid is discharged at once, the polymer sheet cannot quickly absorb the entire body fluid, and the amount of backflow of the body fluid to the surface increases.

Various improvements have been made on such absorbent articles, and technologies for improving the absorption capability of the polymer sheet have been proposed. For example, Patent Document 1 discloses a body-fluid absorbing article including an absorber that includes a sealed bag partitioned into multiple compartments and superabsorbent polymer particles that are movably enclosed in the compartments of the bag. The absorber is configured such that the partitions are removed when the superabsorbent polymer particles absorb a body fluid and swell.

Patent Document 2 discloses an absorbent article including an absorber that includes a first absorbing layer including a superabsorbent polymer (A) whose fluid permeation time is less than or equal to 20 seconds and a second absorbing layer including a superabsorbent polymer (B) whose saline absorption rate according to the DW method is greater than or equal to 5 g / 30 seconds / 0.3 g. The absorbent article is configured such that the superabsorbent polymer (A) and the superabsorbent polymer (B) do not substantially mix with each other, the first absorbing layer is disposed closer to an upper-side sheet, and the second absorbing layer is disposed closer to a back-side sheet.

### [RELATED-ART DOCUMENTS]

### [Patent Documents]

[Patent Document 1] Japanese Laid-Open Patent Publication No. 2003-265525
[Patent Document 2] Japanese Laid-Open Patent Publication No. H10-118117

Further documents US 5 281 207 A, JP 2008/284190 A, WO 2007/035038 A1, US 2012/136329 A1, EP 2 907 493 A1, JP 2010 088528 A, WO 2013/099634 A1, WO 2011 /043256 A1 and JP 2015 066382 A relate to absorbent articles. Further, reference may be made to EP 2 486 901 A1, which relates to a water-absorbent sheet

### DISCLOSURE OF INVENTION

### PROBLEMS TO BE SOLVED BY THE INVENTION

In Patent Document 1, the partitions are removed when the superabsorbent polymer particles absorb a body fluid and swell so that the polymer particles can freely swell beyond the limitation of the compartments and gel blocking due to adhesion of the polymer particles is prevented. In Patent Document 2, the fluid permeation time of the superabsorbent polymer (A) of the first absorption layer is set at a value less than or equal to a predetermined time, and the water absorption rate of the superabsorbent polymer (B) of the second absorption layer is set at a value greater than or equal to a predetermined rate to prevent gel blocking.

In such a polymer sheet, the diffusion of fluid in the plane direction is hardly expected in the superabsorbent polymer layer, and is largely caused by an upper layer sheet and a lower layer sheet covering the skin side and the non-skin side of the superabsorbent polymer layer. Here, the upper layer sheet is generally made of a material that does not tend to retain a fluid and has high fluid permeability to prevent a fluid from remaining in the skin side and flowing back to the skin side. Therefore, it is not appropriate to give the upper layer sheet a function to diffuse a fluid in the plane direction. Accordingly, it is necessary to give the lower layer sheet a certain degree of fluid retaining capability and fluid diffusivity in the plane direction.

However, in Patent Documents 1 and 2, only the use of a normal hydrophilic nonwoven fabric or absorbent paper as the lower layer sheet is described, and the degree of diffusion capability is not considered. Accordingly, with the related-art technologies, a body fluid discharged into an area corresponding to a body fluid discharge part tends to remain in the area, the superabsorbent polymer present in the area absorbs a large amount of the body fluid and swells greatly, and gel blocking significantly decreases the absorption capability.

Also, there is often a case where it is not possible to replace an absorbent article with a new one after a first discharge of a body fluid, and the body fluid is repeatedly discharged into the same absorbent article. The most common number of times that a body fluid is repeatedly discharged into the same absorbent article is two. Accordingly, there is a demand for an absorbent article whose absorption capability is less likely to be influenced by gel blocking and that can absorb the body fluid even at a second discharge of the body fluid.

Accordingly, a primary object of the present invention is to provide an absorbent article where the influence of gel blocking on the absorption capability is reduced by increasing the fluid diffusivity on the non-skin side of the absorber to enable the absorbent article to absorb the body fluid at a predetermined absorption rate even at the second discharge of the body fluid.

### MEANS FOR SOLVING THE PROBLEMS

The inventor of the present invention conducted intensive research on the factors that influence the absorption rate of the absorbent article at the second time, and found out that the fluid diffusivity of a non-skin side layer of the absorber is a major factor. Specifically, the inventor has found out that a first discharge of a body fluid can be diffused widely and absorbed/retained in a wide area of the superabsorbent polymer by making the fluid diffusivity of a nonwoven fabric disposed on the non-skin side of the absorber greater than or equal to a predetermined value; and as a result, the absorption capability of the superabsorbent polymer in the area corresponding to the body fluid discharge part is left to absorb a second discharge of the body fluid, and the absorption rate of the second discharge of the body fluid is increased.

Also, the inventor repeated tests to determine the second time absorption rate at which the influence of gel blocking on the absorption capability can be minimized. The inventor has found out that the influence of gel blocking on the absorption capability can be reliably reduced and the body fluid can be absorbed satisfactorily when the absorption rate of the absorber measured according to a predetermined test method is less than or equal to 42 seconds. Accordingly, a nonwoven fabric disposed on the non-skin side of the absorber needs to have such a fluid diffusivity that the absorption rate of the absorber becomes less than or equal to 42 seconds.

To satisfy the above requirements, claim 1 of the present invention provides an absorbent article including at least an absorber. The absorber includes a nonwoven fabric disposed on a non-skin side and having a fluid diffusivity, the nonwoven fabric being formed by stacking a spunbonded layer, a meltblown layer, and a spunbonded layer in this order and including a hydrophilic agent. The absorber is a polymer sheet that includes an upper layer sheet disposed on a skin side, a lower layer sheet disposed on the non-skin side, and a superabsorbent polymer disposed between the upper layer sheet and the lower layer sheet. The lower layer sheet is the nonwoven fabric. The upper layer sheet is formed of a hydrophobic nonwoven fabric including chemical fibers. The nonwoven fabric is configured such that a diffusion area of the nonwoven fabric is greater than or equal to 1500 mm². The diffusion area is measured by an absorption test method including (1) preparing a specimen with a size of 100 mm x 100 mm, (2) placing a tip of a buret at a height of 10 mm above a surface of the specimen, and dropping one droplet of ion-exchanged water from the buret, and (3) after 3 minutes from a time when the droplet reaches the surface of the specimen, measuring, as the diffusion area, an area of a region of the surface of the specimen where a reflection from water is clearly visible.

### ADVANTAGEOUS EFFECT OF THE INVENTION

As described above, according to the present invention, the fluid diffusivity of the non-skin side of an absorber is increased so that the absorber can absorb even a second discharge of a body fluid at a predetermined absorption rate, and the influence of gel blocking on the absorption capability can be reduced.

Embodiments of the present invention are described below.

### (EMBODIMENT 1)

An embodiment 1 provides an absorbent article that includes at least an absorber. The absorber includes a nonwoven fabric disposed on a non-skin side and having a fluid diffusivity, and a diffusion area of the nonwoven fabric is greater than or equal to 1500 mm². The diffusion area is measured by an absorption test method including:
(1) preparing a specimen with a size of 100 mm x 100 mm,
(2) placing a tip of a buret at a height of 10 mm above a surface of the specimen, and dropping one droplet of ion-exchanged water from the buret, and
(3) after 3 minutes from a time when the droplet reaches the surface of the specimen, measuring, as the diffusion area, an area of a region of the surface of the specimen where a reflection from water is clearly visible.

In the embodiment 1, the nonwoven fabric disposed on the non-skin side of the absorber and having a fluid diffusivity has a diffusion area greater than or equal to 1500 mm² that is measure by a predetermined absorption test method. With this configuration, a body fluid, which has penetrated into the absorbent article and has been absorbed by the nonwoven fabric on the non-skin side, is quickly and widely diffused by the nonwoven fabric, and then absorbed and retained in a wide area of the superabsorbent polymer. This in turn prevents or suppresses the discharged body fluid from remaining in an area corresponding to a body fluid discharge part. Thus, increasing the fluid diffusivity on the non-skin side of the absorber enables the absorbent article to absorb even a second discharge of a body fluid at a predetermined absorption rate, and makes it possible to reduce the influence of gel blocking on the absorption capability.

### (EMBODIMENT 2)

According to embodiment 2 the absorption rate of the absorber at the second time is less than or equal to 42 seconds. The absorption rate is measured by an absorption test method including:
(1) after three minutes from a time when 1 cc of artificial menstrual blood is injected into a surface, injecting 1 cc of the artificial menstrual blood into a same position on the surface; and
(2) measuring, as the absorption rate, a time between a second injection of the artificial menstrual blood and completion of absorption of the whole artificial menstrual blood.

According to the embodiment 2, the second time absorption rate measured by a predetermined absorption test method, which is adapted for absorption of a second discharge of the body fluid, is less than or equal to 42 seconds. This configuration makes it possible to reduce the influence of gel blocking by the superabsorbent polymer on the absorption capability, and to obtain an absorbent article configured to prevent the body fluid from remaining on the surface and flowing back to the surface. If the second time absorption rate is greater than 42 seconds, the wearer may feel discomfort such as stickiness due to the body fluid remaining on the surface, and the body fluid tends to flow over the surface and leak from the absorbent article. Also, the backflow of the body fluid from the absorber may cause stickiness and leakage. The second time absorption rate can be made less than or equal to 42 seconds by providing a nonwoven fabric, which has a diffusion area of 1500 mm² or greater measured by the predetermined test method, as the non-skin side layer of the absorber.

### (EMBODIMENT 3)

According to embodiment 3 the nonwoven fabric is formed by stacking a spunbonded layer, a meltblown layer, and a spunbonded layer in this order, and includes a hydrophilic agent.

In the embodiment 3, to achieve the above diffusion area, the nonwoven fabric is formed by stacking a spunbonded layer, a meltblown layer, and a spunbonded layer in this order, and includes a hydrophilic agent. The surfaces of the SMS nonwoven fabric are formed of the spunbonded layers, and the middle portion of the nonwoven fabric is formed of the meltblown layer. This structure enables the nonwoven fabric to reliably retain a body fluid. Also, because the nonwoven fabric includes a hydrophilic agent, the nonwoven fabric can efficiently draw the body fluid by capillary action and has improved fluid diffusivity. A hydrophilic agent may be included in the nonwoven fabric by, for example, a coating method or an impregnation method.

### (EMBODIMENT 4)

According to embodiment 4 the absorber is a polymer sheet that includes an upper layer sheet disposed on a skin side, a lower layer sheet disposed on the non-skin side, and a superabsorbent polymer disposed between the upper layer sheet and the lower layer sheet; and the lower layer sheet is the nonwoven fabric.

The embodiment 4 is a first example where the absorber is implemented by a polymer sheet, and the nonwoven fabric having a predetermined diffusion capability is used as the lower layer sheet of the polymer sheet. Because the superabsorbent polymer is provided as a single mass in the polymer sheet, using a nonwoven fabric having a predetermined diffusion capability as the lower layer sheet makes it possible to improve the effect of reducing the influence of gel blocking.

### (EMBODIMENT 5)

According to embodiment 5 the upper layer sheet is formed of an air-through nonwoven fabric including chemical fibers.

In the embodiment 5, the upper layer sheet of the polymer sheet implementing the absorber is formed of an air-through nonwoven fabric including chemical fibers. With this configuration, the fluid retention capability of the upper layer sheet is reduced, and the body fluid can more smoothly pass through the upper layer sheet. This in turn makes it possible to prevent the body fluid from remaining on the surface and flowing back to the surface, and makes it possible to improve the absorption capability.

### (EMBODIMENT 6)

According to embodiment 6 the polymer sheet includes a middle sheet disposed between the upper layer sheet and the lower layer sheet; the superabsorbent polymer is disposed in at least one of spaces formed between adjacent ones of the upper layer sheet, the lower layer sheet, and the middle sheet; and instead of a configuration where the diffusion area of the lower layer sheet measured by the absorption test method is greater than or equal to 1500 mm², the absorbent article has a configuration where a diffusion area of one or both of the lower layer sheet and the middle sheet measured by the absorption test method is greater than or equal to 1500 mm².

In the embodiment 6, when the absorber is implemented by a polymer sheet, a middle sheet is disposed between the upper layer sheet and the lower layer sheet, and the superabsorbent polymer is disposed in at least one of spaces formed between adjacent ones of the upper layer sheet, the lower layer sheet, and the middle sheet. In the polymer sheet with this structure, a diffusion area of one or both of the lower layer sheet and the middle sheet measured by the absorption test method is preferably greater than or equal to 1500 mm². Preferably, the superabsorbent polymer is disposed in each of a space between the upper layer sheet and the middle sheet and a space between the middle sheet and the lower layer sheet. In this case, the middle sheet is preferably implemented by an air-through nonwoven fabric including chemical fibers, and the lower layer sheet preferably has a diffusion area of 1500 mm² or greater that is measured by the absorption test method. With this configuration, the superabsorbent polymer is divided by the middle sheet into an upper superabsorbent polymer layer and a lower superabsorbent polymer layer. This configuration makes it possible to suppress gel blocking that may occur when the superabsorbent polymer swells as a result of absorbing a fluid.

### (EMBODIMENT 7)

According to embodiment 7 the absorber includes an absorber body that is a mixture of pulp and a superabsorbent polymer, and the nonwoven fabric is disposed on the non-skin side of the absorber body.

The embodiment 7 is a second example where the absorber includes an absorber body that is a mixture of pulp and a superabsorbent polymer, and the nonwoven fabric is disposed on the non-skin side of the absorber body. Due to the fluid diffusivity of the nonwoven fabric, a widely diffused body fluid is absorbed and retained in the absorber body.

### BRIEF DESCRIPTION OF THE DRAWINGS

FIG. 1 is a partially-cutaway development view of a sanitary napkin 1 according to the present invention;
FIG. 2 is a cross-sectional view taken along line II-II of FIG. 1;
FIG. 3 is an enlarged cross-sectional view of a polymer sheet 4;
FIG. 4 is a cross-sectional view illustrating an absorbing state of a related-art polymer sheet;
FIG. 5 is a cross-sectional view illustrating an absorbing state of the polymer sheet 4;
FIG. 6 is a side view of an absorption test apparatus;
FIG. 7 is a cross-sectional view of a variation of the polymer sheet 4;
FIG. 8 is a cross-sectional view of a variation of the polymer sheet 4;
FIG. 9 is a cross-sectional view of an absorber according to a second embodiment; and
FIG. 10 is a graph indicating the results of absorption tests.

### DESCRIPTION OF EMBODIMENTS

Embodiments of the present invention are described below with reference to the accompanying drawings. In the descriptions below, a sanitary napkin is used as an example of an absorbent article.

### <<FIRST EMBODIMENT>>

### <BASIC STRUCTURE OF SANITARY NAPKIN 1>

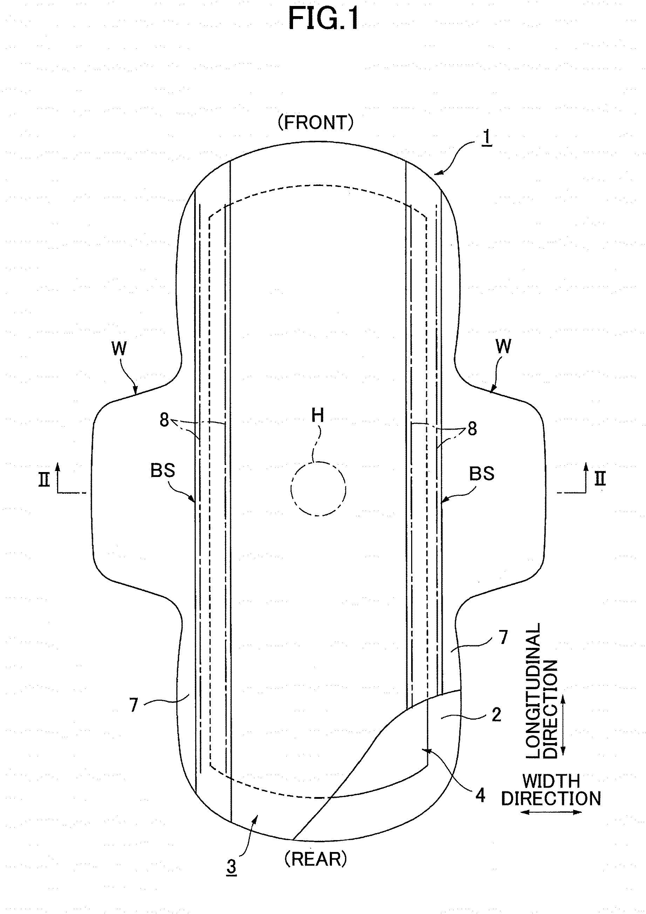

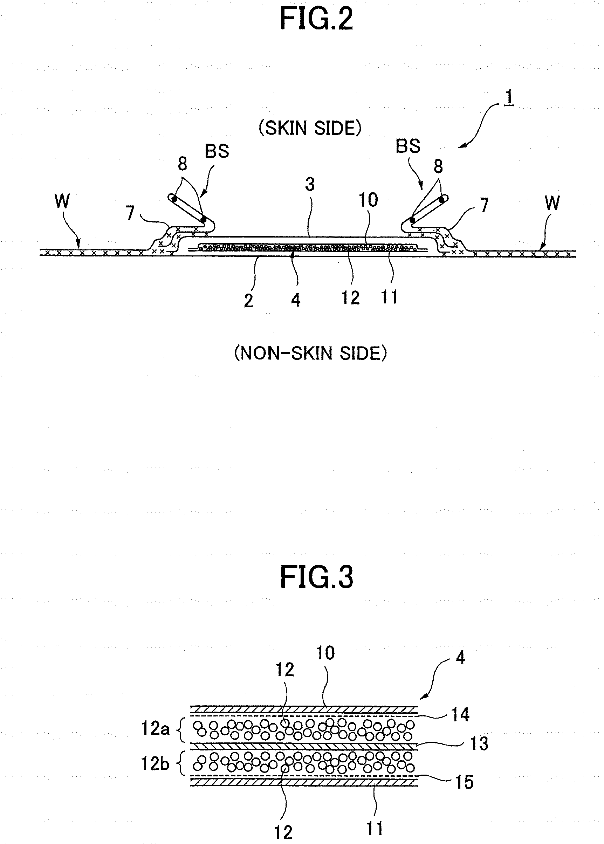

As illustrated in FIGs. 1 and 2, a sanitary napkin 1 according to the present invention includes an impermeable back-side sheet 2 such as a polyethylene sheet; a permeable upper-side sheet 3 that allows, for example, urine to quickly pass therethrough; a polymer sheet 4 that is disposed between the impermeable back-side sheet 2 and the permeable upper-side sheet 3 and includes an upper layer sheet 10 disposed on the skin side, a lower layer sheet 11 disposed on the non-skin side, and a superabsorbent polymer 12 disposed in a predetermined area between the upper layer sheet 10 and the lower layer sheet 11; and side nonwoven fabrics 7 forming a pair of right and left three-dimensional gathers BS that rise from substantially the side edges of the polymer sheet 4 and protrude toward the skin in a predetermined area extending in the longitudinal direction and including at least a body fluid discharge part H. In the periphery of the polymer sheet 4, at the edges in the longitudinal direction of the polymer sheet 4, the outer edges of the impermeable back-side sheet 2 and the permeable upper-side sheet 3 are bonded together with an adhesive such as a hot melt or a bonding method such as heat sealing. At the side edges of the polymer sheet 4, the impermeable back-side sheet 2 extending laterally beyond the polymer sheet 4 and the side nonwoven fabrics 7 are bonded together with an adhesive such as a hot melt or a bonding method such as heat sealing. As necessary, a hydrophilic second sheet (not shown) may be provided between the permeable upper-side sheet 3 and the polymer sheet 4.

The structure of the sanitary napkin 1 is described in more detail below. The impermeable back-side sheet 2 may be implemented by a sheet material such as polyethylene or polypropylene that has at least a water shielding property. Also, a nonwoven fabric sheet that is made practically impermeable by using a waterproof film may be used (in this case, the impermeable back-side sheet is formed by a combination of a waterproof film and a nonwoven fabric). In recent years, materials with moisture permeability are preferably used to prevent stuffiness. The water-shielding/moisture-permeable sheet material is preferably a microporous sheet that is obtained by forming a sheet by melt-kneading a mixture of an inorganic filler and an olefinic resin such as polyethylene or polypropylene, and stretching the sheet in a uniaxial or biaxial direction.

The permeable upper-side sheet 3 is preferably formed of, for example, a porous or non-porous nonwoven fabric or a porous plastic sheet. Examples of fiber materials of the nonwoven fabric include synthetic fibers made of olefin such as polyethylene or polypropylene, polyester, or polyamide; regenerated fibers such as rayon and cupra, and natural fibers such as cotton. The nonwoven fabric may be produced by any appropriate production method such as spunlacing, spunbonding, thermal bonding, melt blowing, or needle punching. Among these production methods, spunlacing is preferable in terms of flexibility and draping characteristics, and thermal bonding is preferable to produce a soft and bulky nonwoven fabric.

The side nonwoven fabrics 7 are provided on the upper lateral sides of the sanitary napkin 1 and extend along the entire length of the sanitary napkin 1 in the longitudinal direction. The outer portions of the side nonwoven fabrics 7 extend laterally, and the impermeable back-side sheet 2 also extends laterally. The laterally-extending portions of the side nonwoven fabrics 7 and the impermeable back-side sheet 2 are bonded together with, for example, a hot melt adhesive to form side flaps.

The side nonwoven fabric 7 may be implemented by either a water-repellent nonwoven fabric or a hydrophilic nonwoven fabric depending on the desired function. For example, when priority is given to a function for preventing permeation of menstrual blood or a function for improving the feel is important, a water-repellent nonwoven fabric such as SSMS (a nonwoven fabric formed by stacking a spunbonded layer, a spunbonded layer, a meltblown layer, and a spunbonded layer sequentially), SMS, or SMMS (a nonwoven fabric formed by stacking a spunbonded layer, a meltblown layer, a meltblown layer, and a spunbonded layer sequentially) coated with a silicon-, paraffin-, or alkyl chromic chloride- water-repellent agent is preferably used. When priority is given to the absorbency of a body fluid, a hydrophilic nonwoven fabric is preferably used. For example, such a hydrophilic nonwoven fabric may be prepared by polymerizing a compound having a hydrophilic group such as an oxidation product of polyethylene glycol during the production process of synthetic fibers. Also, a hydrophilic nonwoven fabric may be prepared by processing synthetic fibers with metal salt such as stannic chloride to partially dissolve the surfaces of the synthetic fibers and give them porosity, and by depositing metal hydroxide on the synthetic fibers. The resulting synthetic fibers become swollen or porous and exhibit a hydrophilic property due to capillarity. The side nonwoven fabrics 7 may be formed by processing natural fibers, synthetic fibers, or regenerated fibers using any appropriate method.

The side nonwoven fabrics 7 are folded appropriately to form a pair of right and left three-dimensional gathers BS that rise toward the skin from positions near the side edges of the absorber 4.

### <POLYMER SHEET 4>

The polymer sheet 4 is described in detail below. As illustrated in FIG. 3, the polymer sheet 4 disposed between the impermeable back-side sheet 2 and the permeable upper-side sheet 3 has a structure where the superabsorbent polymer 12 is disposed between the upper layer sheet 10 disposed on the skin side (on the side of the permeable upper-side sheet 3) and the lower layer sheet 11 disposed on the non-skin side (on the side of the impermeable back-side sheet 2). The superabsorbent polymer 12 is not dispersed in a fibrous material such as pulp between the upper layer sheet 10 and the lower layer sheet 11, but is provided alone (or independently) as an aggregate of particles of the superabsorbent polymer 12. This configuration makes it possible to reduce the thickness of the polymer sheet 4 and reduce the thickness of the sanitary napkin 1.

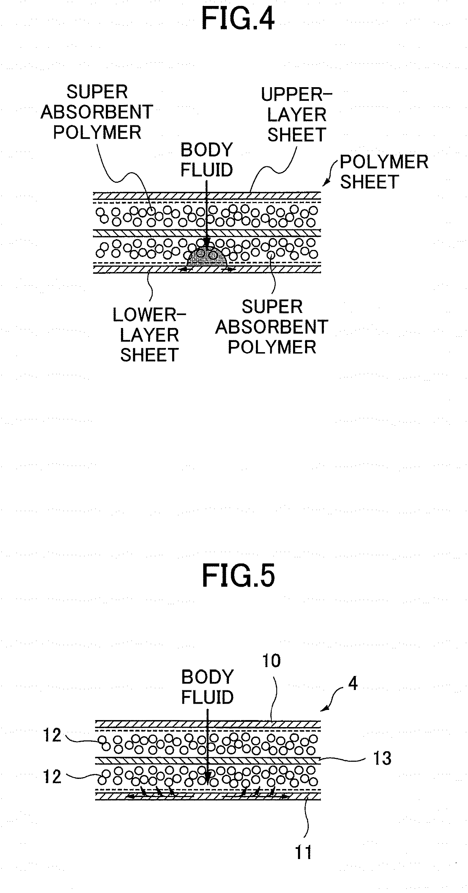

In the sanitary napkin 1, to enable the polymer sheet 4 to absorb the second discharge of the body fluid at an absorption rate that does not cause gel blocking, the lower layer sheet 11 of the polymer sheet 4 has a diffusivity with a diffusion area greater than or equal to a predetermined diffusion area. In a related-art polymer sheet, as illustrated in FIG. 4, the diffusivity of the lower-layer sheet is not considered, and therefore the body fluid penetrated into the polymer sheet tends to remain in the area corresponding to the body fluid discharge part and is preferentially absorbed and retained by the superabsorbent polymer in this area. When the absorption capability of the superabsorbent polymer in this area is saturated, the body fluid flows through gaps between superabsorbent polymer particles into the surrounding superabsorbent polymer and is absorbed by the surrounding superabsorbent polymer. Thus, in the related-art polymer sheet, the absorption area gradually increases. Accordingly, at the second discharge of the body fluid, the absorption capability of the superabsorbent polymer in the area corresponding to the body fluid discharge part is already saturated, and gel blocking tends to occur due to the swelled superabsorbent polymer particles adhering to each other. On the other hand, in the polymer sheet 4 according to the present invention, as illustrated in FIG. 5, the lower layer sheet 11 has such a diffusivity that the diffusion area becomes greater than or equal to a predetermined value. With this configuration, at the first discharge of the body fluid, the body fluid is diffused as far as possible into areas outside of the area corresponding to the body fluid discharge part before the superabsorbent polymer in this area is saturated, and the absorption capacity of the superabsorbent polymer in the area can be left. Therefore, the second discharge of the body fluid can also be absorbed by the superabsorbent polymer in the area corresponding to the body fluid discharge part, and can be diffused by the lower layer sheet 11 and absorbed by a wider area of the superabsorbent polymer. Thus, the polymer sheet 4 has a structure that is excellent in absorption capability and that does not cause gel blocking even at the second discharge of the body fluid.

A porous or non-porous nonwoven fabric is used as the upper layer sheet 10 that constitutes the skinside layer of the polymer sheet 4. A hydrophobic fiber material including chemical fibers is preferably used for the nonwoven fabric. Specifically, similarly to the permeable upper-side sheet 3, examples of fiber materials include thermoplastic synthetic fibers made of, for example, olefin such as polyethylene or polypropylene, polyester, or polyamide; and a mixture of these synthetic fibers and regenerated fibers such as rayon or cupra and/or natural fibers such as cotton. The nonwoven fabric may be formed by any processing method. However, to reduce the fluid retention capability of the nonwoven fabric, a processing method such as an air-through method, with which the fiber density of an obtained product becomes low, is preferably used. Alternatively, to prevent the superabsorbent polymer 12 from dropping off, a processing method such as spunbonding, meltblowing, or needle punching, with which the fiber density of an obtained product becomes high, may be used. When the upper layer sheet 10 includes chemical fibers, the fluid retention capability of the upper layer sheet 10 is reduced, the body fluid can more smoothly pass through the upper layer sheet 10 toward the lower layer, and the backflow of the body fluid retained in the upper layer sheet 10 toward the skin is suppressed.

The mass per unit area of the upper layer sheet 10 is preferably between 15 and 40 g/m² and more preferably between 18 and 30 g/m², and the thickness of the upper layer sheet 10 is preferably between 0.05 and 0.4 mm and more preferably between 0.14 and 0.23 mm. The mass per unit area is measured by cutting a portion with a size of 20 mm x 40 mm (±2 mm) from a sample using a roll cutter, measuring the weight of the cut portion, and converting the measured weight into a weight per 1 m² (g/m²). The thickness is measured using a thickness measuring device (PEACOCK, dial thickness gauge: large, model J-B (measurement range: 0 to 35 mm)) of OZAKI MFG. Co., Ltd. with the sample and thickness measuring device disposed horizontally. Mass per unit areas and thicknesses described below may also be measured similarly.

To make the absorption rate of the polymer sheet 4 at the second time less than or equal to a predetermined value, the diffusion area of the lower layer sheet 11 measured by an absorption test method described below is greater than or equal to 1500 mm², preferably between 1500 and 2500 mm², and more preferably between 2000 and 2500 mm². The absorption test method described below is based on JIS L 1907. Details of the test not described below are based on this standard.

### <ABSORPTION TEST METHOD>

(1) Prepare a specimen with a size of 100 x 100 mm.
(2) Place the tip of a buret at a height of 10 mm above the surface of the specimen, and drop one droplet of ion-exchanged water from the buret.
(3) After 3 minutes from the time when the droplet reaches the surface of the specimen, measure, as the diffusion area, an area of a region of the surface of the specimen where a reflection from water is clearly visible.

The diffusion area is measured by capturing image data of the surface of the specimen into a computer, specifying the outline of a diffusion region, and analyzing the area of the enclosed region using image analysis software.

The sheet material having a diffusion area greater than or equal to 1500 mm² measured by the above test method is preferably a nonwoven fabric (SMS nonwoven fabric) that is formed by stacking a spunbonded layer, a meltblown layer, and a spunbonded layer in this order and includes a hydrophilic agent. Particularly, a superhydrophilic SMS nonwoven fabric manufactured by Golden Phoenix is preferably used.

The SMS nonwoven fabric has a structure where the upper surface is formed of a spunbonded layer and the middle layer is formed of meltblown layer. With this structure, a body fluid can readily penetrate from a spunbonded layer with a relatively low density into the meltblown layer with a relatively high density, and can be reliably retained in the SMS nonwoven fabric. Also, because the SMS nonwoven fabric includes a hydrophilic agent, the SMS nonwoven fabric can efficiently draw the body fluid by capillary action and has improved fluid diffusivity in the plane direction. When an air-through nonwoven fabric is used as the lower layer sheet, even if a hydrophilic agent is applied to the air-through nonwoven fabric, the capability of the air-through nonwoven fabric to draw a fluid by the capillary action is low because of its low density. Accordingly, the fluid passes through and flows out of the lower layer sheet in the thickness direction, and cannot diffuse widely in the plane direction Also, compared with a nonwoven fabric, absorbent paper such as crepe paper is more easily torn when the absorbent paper is embossed, and the strength of wet absorbent paper that has absorbed a fluid is low. Accordingly, the absorbent paper tends to be torn by a pressure generated when the superabsorbent polymer absorbs a fluid and swells or by the movement of the body of the wearer. Accordingly, using absorbent paper may increase the risk that the superabsorbent polymer drops off.

A hydrophilic agent may be included in the lower layer sheet 11 by, for example, a coating method or an impregnation method. Examples of coating methods include spraying, gravure printing, flexographic printing, and curtain coating using a coater. In the impregnation method, the SMS nonwoven fabric is immersed in a solution of a hydrophilic agent. The degree of hydrophilicity can be adjusted by adjusting the content of the hydrophilic agent.

Examples of hydrophilic agents include an anionic surfactant, carboxylate, acylated hydrolyzed protein, sulfonate, sulfate ester, phosphate ester, a nonionic surfactant, a polyoxyethylene-based surfactant, carboxylate ester, carboxylic amide, a polyalkylenoxide block copolymer, a cationic surfactant, quaternary ammonium salt, an amphoteric surfactant, and imidazolinium derivative. Also, any other known hydrophilic agent that can be applied to fibers may be used.

The diffusion area of the lower layer sheet 11 may be 1.5 to 3 times and preferably 2 to 2.5 times greater than the diffusion area of an air-through nonwoven fabric (the upper layer sheet 10). Also, the diffusion area of the lower layer sheet 11 may be 1.1 to 2.2 times and preferably 1.4 to 1.8 times greater than the diffusion area of crepe paper.

The lower layer sheet 11 is preferably implemented by a nonwoven fabric having a fiber density that is higher than the fiber density of the upper layer sheet 10. This enables the lower layer sheet 11 to more effectively retain a fluid, and facilitates the diffusion of the fluid in the plane direction by the capillary action. The mass per unit area of the lower layer sheet 11 may be 1.2 to 3 times and preferably 1.5 to 2 times greater than the mass per unit area of the upper layer sheet 10.

The second time absorption rate of the polymer sheet 4 with the above configuration is less than or equal to 42 seconds, preferably between 30 seconds and 42 seconds, and more preferably between 30 seconds and 35 seconds, and is measured by an absorption test method described below.

### <ABSORPTION TEST METHOD>

(1) After three minutes from the time when 1 cc of artificial menstrual blood is injected into the surface of a specimen formed by stacking an air-through nonwoven fabric of 20 g/m² on the surface of the upper layer sheet 10, 1 cc of artificial menstrual blood is injected into the same position on the surface of the specimen. The artificial menstrual blood is composed of 12.30 weight percent of glycerin, 85.18 weight percent of ion-exchanged water, 0.45 weight percent of CMC (sodium carboxymethylcellulose), 0.97 weight percent of NaCl (sodium chloride), 1.04 weight percent of Na₂CO₃ (sodium carbonate), and 0.06 weight percent of blue powder, and is prepared to have a viscosity of 8 mPa▪ s, 37°C.
(2) The time between the second injection of the artificial menstrual blood and the completion of the absorption of the whole artificial menstrual blood is measured with a stopwatch to obtain a second time absorption rate.

The second time absorption rate of the polymer sheet 4 measured by the above absorption test method can be made less than or equal to 42 seconds by making the diffusion area of the lower layer sheet 11 measured by the predetermined method greater than or equal to 1500 mm².

The mass per unit area of the lower layer sheet 11 may be between 15 and 40 g/m² and preferably between 20 and 35 g/m², and the thickness of the lower layer sheet 11 may be between 0.05 and 0.3 mm and preferably between 0.08 and 0.12 mm.

Although any appropriate material may be used for the superabsorbent polymer 12, a material with an absorption capacity greater than or equal to 40 g/g is preferably used. Examples of the superabsorbent polymer 12 include crosslinked polyacrylate, self-crosslinked polyacrylate, saponified product of crosslinked acrylate-vinyl acetate copolymer, crosslinked isobutylene-maleic anhydride copolymer, crosslinked polysulfonate, and partially-crosslinked water-swellable polymer such as polyethylene oxide or polyacrylamide. Among them, polymers including acrylic acid or acrylate are preferable in terms of the absorption capacity and the absorption rate. The absorption power and the absorption rate of the superabsorbent polymer with the above absorption capability can be adjusted by adjusting the crosslink density and the crosslink density gradient during the production process.

As illustrated in FIG. 3, the superabsorbent polymer 12 is preferably bonded to the upper layer sheet 10 and the lower layer sheet 11 via adhesive layers 14 and 15, which are made of, for example, a hot melt adhesive and are provided, respectively, between the superabsorbent polymer 12 and the upper and lower layer sheets 10 and 11.

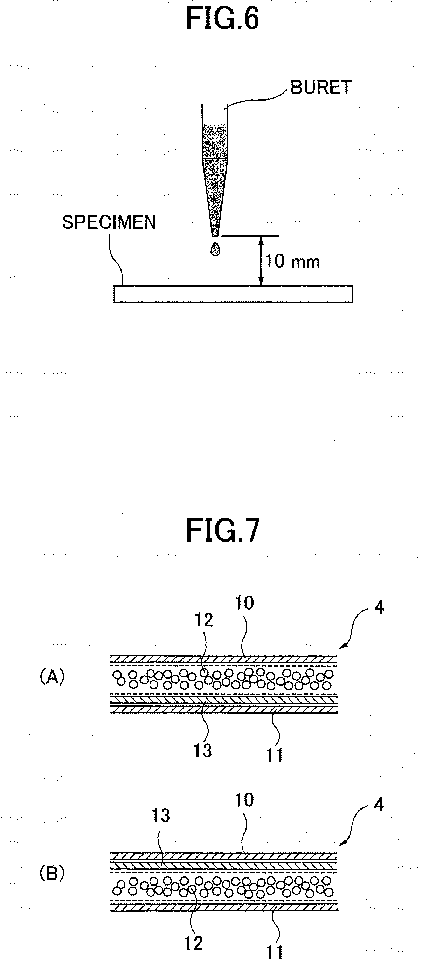

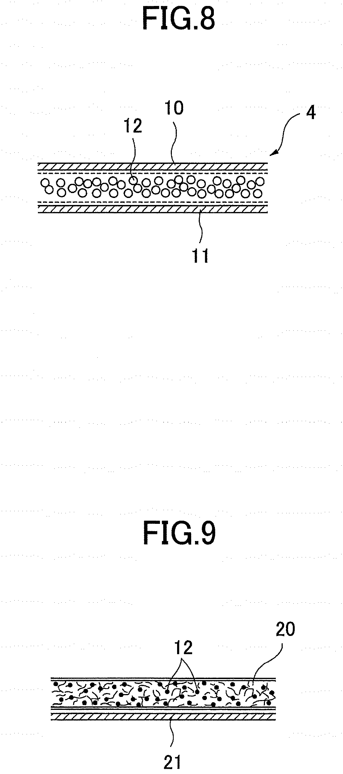

The polymer sheet 4 preferably includes a middle sheet 13 disposed between the upper layer sheet 10 and the lower layer sheet 11, and the superabsorbent polymer 12 is preferably provided at least in one space between the upper layer sheet 10, the lower layer sheet 11, and the middle sheet 13 (i.e., at least one of the space between the upper layer sheet 10 and the middle sheet 13 and the space between the middle sheet 13 and the lower layer sheet 11). In the example illustrated in FIG. 3, the superabsorbent polymer 12 is disposed between the upper layer sheet 10 and the middle sheet 13 and between the middle sheet 13 and the lower layer sheet 11 such that a five-layer structure is formed. FIG. 7 (A) illustrates a variation where the superabsorbent polymer 12 is provided only between the upper layer sheet 10 and the middle sheet 13 such that a four-layer structure is formed. FIG. 7 (B) illustrates a variation where the superabsorbent polymer 12 is provided only between the middle sheet 13 and the lower layer sheet 11 such that a four-layer structure is formed. FIG. 8 illustrates still another variation where the middle sheet 13 is not provided and the superabsorbent polymer 12 is provided between the upper layer sheet 10 and the lower layer sheet 11 such that a three-layer structure is formed.

As illustrated in FIG. 3, providing the middle sheet 13 that divides the superabsorbent polymer 12 into two layers makes it possible to prevent the upper-layer superabsorbent polymer 12 and the lower-layer superabsorbent polymer 12, which have swollen as a result of absorbing a fluid, from adhering to each other, and thereby makes it possible to more reliably suppress gel blocking.

The middle sheet 13 may be implemented by a porous or non-porous nonwoven fabric. The fiber material of the nonwoven fabric may be changed depending on its purpose. For example, when layers of the superabsorbent polymer 12 are provided on the upper and lower sides of the middle sheet 13, the middle sheet 13 may be formed of a material that is less likely to retain a fluid and has an excellent permeability. For example, similarly to the upper layer sheet 10, an air-through nonwoven fabric including chemical fibers may be used for the middle sheet 13. Also, the middle sheet 13 may be configured to diffuse a fluid in a plane direction to prevent gel blocking. That is, the polymer sheet 4 may be configured such that a body fluid absorbed by the middle sheet 13 is diffused in the plane direction and is absorbed by the superabsorbent polymer 12 provided on each of the upper side and the lower side of the middle sheet 13 to further increase the absorption rate. Here, similarly to the lower layer sheet 11, the middle sheet 13 may be formed of a nonwoven fabric whose diffusion area is greater than or equal to 1500 mm². However, a large number of openings are preferably formed in the middle sheet 13 at predetermined intervals so that a predetermined amount of fluid can pass through the middle sheet 13 and the permeability of a body fluid into the lower layer side is increased.

Also, with the configuration illustrated in FIG. 7 (A) where the superabsorbent polymer 12 is not provided between the middle sheet 13 and the lower layer sheet 11, a material with a diffusion area greater than or equal to 1500 mm² measured by the above absorption test method is preferably used to give a fluid diffusivity to the middle sheet 13. In this case, the lower layer sheet 11 may be formed of either a nonwoven fabric with a diffusion area greater than or equal to 1500 mm² or any other nonwoven fabric.

Preferably, the diffusion area of the middle sheet 13 is substantially equal to or less than the diffusion area of the lower layer sheet 11. Specifically, the diffusion area of the middle sheet 13 may be 0.5 to 1.0 times and preferably 0.6 to 0.8 times the diffusion area of the lower layer sheet 11. Here, the diffusion area is measured by the absorption test method described above. With this configuration, the body fluid diffused by the middle sheet 13 can be further diffused across a wider area by the lower layer sheet 11, and gel blocking at the second discharge of the body fluid can be more reliably prevented. The diffusion area of each sheet can be adjusted by adjusting the content of the hydrophilic agent.

The mass per unit area of the middle sheet 13 may be between 20 and 40 g/m² and preferably between 25 and 30 g/m², and the thickness of the middle sheet 13 may be between 0.2 and 0.7 mm and preferably between 0.36 and 0.42 mm.

The mass per unit area of a superabsorbent polymer layer 12a on the upper side and the mass per unit area of a superabsorbent polymer layer 12b on the lower side may be substantially the same as illustrated in FIG. 3 or may be different from each other. When they are different from each other, the mass per unit area of the superabsorbent polymer layer 12a may be greater than the mass per unit area of the superabsorbent polymer layer 12b, or the mass per unit area of the superabsorbent polymer layer 12b may be greater than the mass per unit area of the superabsorbent polymer layer 12a. In the former case, the body fluid that has passed through the upper layer sheet 10, the upper superabsorbent polymer layer 12a, and the middle sheet 13 can quickly pass through the lower superabsorbent polymer layer 12b, can be smoothly absorbed by the lower layer sheet 11, and can be efficiently diffused by the lower layer sheet 11. In the latter case, the body fluid diffused by the lower layer sheet 11 can be smoothly absorbed by the lower superabsorbent polymer layer 12b that is adjacent to the lower layer sheet 11.

The mass per unit area of the superabsorbent polymer 12 in the upper superabsorbent polymer layer 12a may be between 10 and 300 g/m² and preferably between 65 and 150 g/m². The mass per unit area of the superabsorbent polymer 12 in the lower superabsorbent polymer layer 12b may be between 0 and 300 g/m² and preferably between 0 and 65 g/m².

The superabsorbent polymer may include "powder" in addition to "particles". Any superabsorbent polymer with a particle diameter suitable for this type of absorbent article may be used without change. The average particle diameter of the superabsorbent polymer is less than or equal to 1000 µm. Preferably, particles having a diameter greater than or equal to 106 µm before fluid absorption constitute 99 weight percent or more of the entire superabsorbent polymer. More preferably, particles with a diameter between 150 µm and 850 µm constitute 99 weight percent or more of the entire superabsorbent polymer. The average particle diameter of the superabsorbent polymer is preferably between about 250 µm and about 500 µm before fluid absorption. Also, the average particle diameter of the superabsorbent polymer after fluid absorption is preferably three times or more greater than the average particle diameter of the superabsorbent polymer before fluid absorption. Specifically, the average particle diameter of the superabsorbent polymer after fluid absorption is preferably greater than or equal to 500 µm. Here, the average particle diameter of the superabsorbent polymer before fluid absorption indicates a particle diameter at a cumulative value of 50% in the weight-based particle size distribution. In this case, the weight-based particle size distribution is measured according to JIS Z8815-1994. In this method, sieves having an inner diameter of 150 mm and a depth of 45 mm and having apertures with diameters of 710 µm, 500 µm, 300 µm, 150 µm, and 106 µm are stacked in descending order of the aperture diameter. Next, 50 g of a measurement sample is placed on the top sieve with the widest aperture diameter of 710 µm and is sieved for 10 minutes using a sieve shaker. Then, the weight of the measurement sample remaining on each sieve is measured, and the weight percent of the measurement sample remaining on each sieve is obtained based on the initial weight of the measurement sample.

### <<SECOND EMBODIMENT>>

In the first embodiment, the polymer sheet 4 is used as the absorber. In a second embodiment, as illustrated in FIG. 9, the absorber includes an absorber body 20 that is a mixture of pulp and the superabsorbent polymer 12, and a nonwoven fabric 21 that is disposed on the non-skin side of the absorber body 20 and has a diffusion area of 1500 mm² or greater that is measured by the absorption test method described above.

The absorber body 20 may be a stacked fiber absorber where the superabsorbent polymer 12 is dispersed in pulp fibers.

The nonwoven fabric 21 has a diffusion area of 1,500 mm² or greater that is measured by the absorption test method described above, and may have a configuration similar to the lower layer sheet 11 of the polymer sheet 4 in the first embodiment.

### «EXAMPLES»

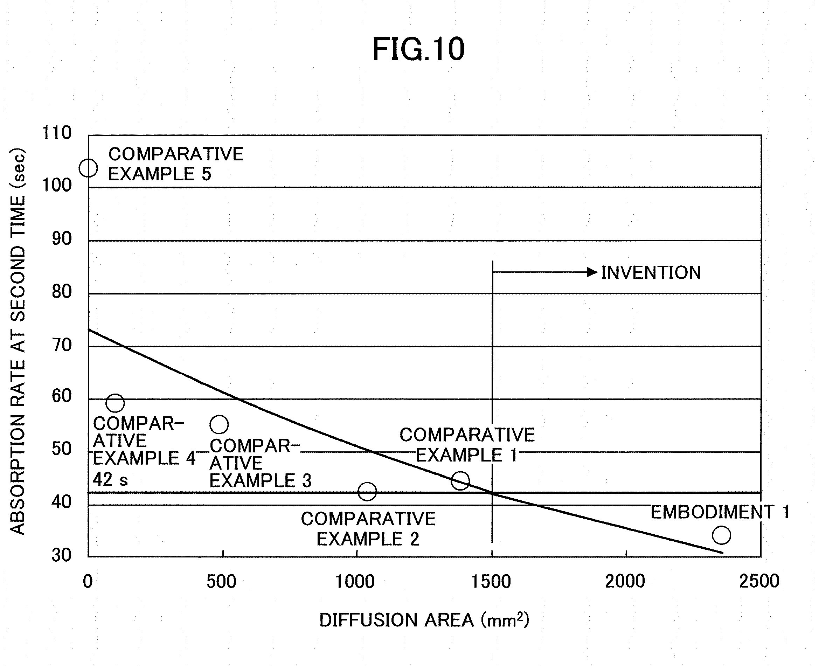

The second time absorption rate of the polymer sheet was measured by the above absorption test method using six types of lower layer sheets 11 with different diffusion areas measured by the above absorption test method. The measurement results are provided in Table 1 and FIG. 10. A polymer sheet with a five-layer structure illustrated in FIG. 3 was used in the measurement. An air-through nonwoven fabric was used as the upper layer sheet 10, and an air-through nonwoven fabric was used as the middle sheet 13.
Embodiment 1:
   SMS nonwoven fabric (superhydrophilic SMS nonwoven fabric manufactured by Golden Phoenix) having a diffusion area of 1500 mm² or greater mass per unit area is 30 g/m²
Comparative Example 1:
   crepe paper
   mass per unit area is 13.5 g/m²
Comparative Example 2:
   hydrophilic SMS nonwoven fabric coated with hydrophilic agent
   mass per unit area is 17 g/m²
Comparative Example 3:
   nonwoven fabric made of 100 weight percent of cotton fibers
   mass per unit area is 33 g/m²
Comparative Example 4:
   air-through nonwoven fabric made of polyethylene and polyethylene terephthalate
   mass per unit area is 20 g/m²
Comparative Example 5:
   hydrophobic SMS nonwoven fabric without hydrophilic treatment
   mass per unit area is 17 g/m²

**[Table 1]**

| | Embodiment 1 | Comparative Example 1 | Comparative Example 2 | Comparative Example 3 | Comparative Example 4 | Comparative Example 5 |
|---|---|---|---|---|---|---|
| Diffusion area (mm²) | 2356 | 1386 | 1037 | 484 | 100 | 0 |
| Second time absorption rate (s) | 34.09 | 44.31 | 42.26 | 55.24 | 59.16 | 103.7 |

A diffusion area of 0 mm² in Comparative Example 5 indicates that the injected ion-exchange water is repelled and does not diffuse, and does not spread over an area greater than or equal to the size of the water drop.

As indicated by Table 1 and FIG. 10, in Embodiment 1 where a lower layer sheet 11 having a diffusion area of 1500 mm² or greater measured by the above absorption test method, the influence of gel blocking on the absorption capability was reduced by diffusing the fluid by the lower layer sheet 11, and the second time absorption rate of the polymer sheet 4 was improved to 42 seconds or shorter. This absorption rate is faster than the absorption rate of crepe paper (Comparative Example 1) that is generally known as a material having a high fluid diffusivity, and the effect of the present invention was proved.

The above-described embodiments can be applied to a sanitary napkin, an incontinence pad, a pantiliner, a medical pad, toiletries, and a disposable diaper.

### EXPLANATION OF REFERENCE NUMERALS

1 ... sanitary napkin, 2 ... impermeable back-side sheet, 3 ... permeable upper-side sheet, 4 ... polymer sheet, 7 ... side nonwoven fabric, 8 ... elastic string, 10 ... upper layer sheet, 11 ... lower layer sheet, 12 ... superabsorbent polymer, 13 ... middle sheet, 14,15 ... adhesive layer

## Claims

1. An absorbent article (1), comprising:
at least an absorber (4) , wherein
the absorber (4) includes a nonwoven fabric (21) disposed on a non-skin side and having a fluid diffusivity, the nonwoven fabric (21) being formed by stacking a spunbonded layer, a meltblown layer, and a spunbonded layer in this order and including a hydrophilic agent;
the absorber (4) is a polymer sheet that includes an upper layer sheet (10) disposed on a skin side, a lower layer sheet (11) disposed on the non-skin side, and a superabsorbent polymer (12) disposed between the upper layer sheet (10) and the lower layer sheet (11) ;
the lower layer sheet (11) is the nonwoven fabric (21) ;
the upper layer sheet (10) is formed of a hydrophobic nonwoven fabric including chemical fibers; and
the nonwoven fabric (21) is configured such that a diffusion area of the nonwoven fabric (21) is greater than or equal to 1500 mm², the diffusion area being measured by an absorption test method including:
(1) preparing a specimen with a size of 100 mm x 100 mm,
(2) placing a tip of a buret at a height of 10 mm above a surface of the specimen, and dropping one droplet of ion-exchanged water from the buret, and
(3) after 3 minutes from a time when the droplet reaches the surface of the specimen, measuring, as the diffusion area, an area of a region of the surface of the specimen where a reflection from water is clearly visible.

2. The absorbent article (1) as claimed in claim 1, wherein an absorption rate of the absorber (4) at a second time is less than or equal to 42 seconds, the absorption rate being measured by an absorption test method including:
(1) after three minutes from a time when 1 cc of artificial menstrual blood is injected into a surface, injecting 1 cc of the artificial menstrual blood into a same position on the surface; and
(2) measuring, as the absorption rate, a time between a second injection of the artificial menstrual blood and completion of absorption of the whole artificial menstrual blood.

3. The absorbent article (1) as claimed in claim 1, wherein the upper layer sheet (10) is formed of a nonwoven fabric including chemical thermoplastic synthetic fibers and formed by an air-through method.

4. The absorbent article (1) as claimed in claim 1, wherein
the polymer sheet includes a middle sheet disposed between the upper layer sheet (10) and the lower layer sheet (11); and
the superabsorbent polymer (12) is disposed in at least one of spaces formed between adjacent ones of the upper layer sheet (10), the lower layer sheet (11), and the middle sheet; and in addition to the lower layer sheet (11), the middle sheet is formed of a nonwoven fabric and a diffusion area of the middle sheet measured by the absorption test method is greater than or equal to 1500 mm².

5. The absorbent article (1) as claimed in claim 1, wherein the absorber (4) includes an absorber body (20) that is a mixture of pulp and a superabsorbent polymer (12), and the nonwoven fabric (21) is disposed on the non-skin side of the absorber body (20).

## Patentansprüche

1. Absorbierender Artikel (1), umfassend:
mindestens einen Absorber (4), wobei
der Absorber (4) einen Vliesstoff (21) umfasst, der auf einer Nicht-Hautseite angeordnet ist und eine Fluid-Diffusionsfähigkeit aufweist, wobei der Vliesstoff (21) durch Stapeln einer Spinnvlies-Schicht, einer Meltblown-Schicht und einer Spinnvlies-Schicht in dieser Reihenfolge gebildet ist und ein hydrophiles Mittel umfasst;
der Absorber (4) eine Polymerfolie ist, die eine obere Schichtlage (10), die auf einer Hautseite angeordnet ist, eine untere Schichtlage (11), die auf der Nicht-Hautseite angeordnet ist, und ein superabsorbierendes Polymer (12), das zwischen der oberen Schichtlage (10) und der unteren Schichtlage (11) angeordnet ist, umfasst;
die untere Schichtlage (11) ist der Vliesstoff (21) ;
die obere Schichtlage (10) aus einem hydrophoben Vliesstoff mit chemischen Fasern gebildet ist; und
der Vliesstoff (21) so konfiguriert ist, dass eine Diffusionsfläche des Vliesstoffs (21) größer als oder gleich 1500 mm2 ist, wobei die Diffusionsfläche durch ein Absorptionstestverfahren gemessen wird, das Folgendes umfasst:
(1) Vorbereiten einer Probe mit einer Größe von 100 mm x 100 mm,
(2) Anbringen einer Bürettenspitze in einer Höhe von 10 mm über einer Oberfläche der Probe und Eintropfen eines Tropfens ionenausgetauschten Wassers aus der Bürette, und
(3) nach 3 Minuten ab dem Zeitpunkt, zu dem der Tropfen die Oberfläche der Probe erreicht, Messung der Fläche eines Bereichs der Oberfläche der Probe, in dem eine Reflexion von Wasser deutlich sichtbar ist, als Diffusionsfläche.

2. Absorbierender Gegenstand (1) nach Anspruch 1, wobei eine Absorptionsrate des Absorbers (4) zu einem zweiten Zeitpunkt kleiner oder gleich 42 Sekunden ist, wobei die Absorptionsrate durch ein Absorptionstestverfahren gemessen wird, das Folgendes umfasst:
(1) nach drei Minuten ab dem Zeitpunkt, zu dem 1 cm³ künstliches Menstruationsblut in eine Oberfläche injiziert wird, Injektion von 1 cm³ des künstlichen Menstruationsblutes in dieselbe Position auf der Oberfläche; und
(2) Messen der Zeit zwischen einer zweiten Injektion des künstlichen Menstruationsblutes und dem Abschluss der Absorption des gesamten künstlichen Menstruationsblutes als Absorptionsrate.

3. Absorbierender Artikel (1) nach Anspruch 1, wobei die obere Schichtlage (10) aus einem Vliesstoff gebildet ist, der chemische thermoplastische synthetische Fasern enthält und durch ein Luftdurchgangsverfahren gebildet ist.

4. Absorbierender Artikel (1) nach Anspruch 1, wobei
die Polymerlage eine mittlere Lage umfasst, die zwischen der oberen Schichtlage (10) und der unteren Schichtlage (11) angeordnet ist; und
das superabsorbierende Polymer (12) in mindestens einem der Räume angeordnet ist, die zwischen benachbarten Schichten der oberen Schichtlage (10), der unteren Schichtlage (11) und der mittleren Schichtlage gebildet sind; und zusätzlich zu der unteren Schichtlage (11) die mittlere Schicht aus einem Vliesstoff gebildet ist und eine durch das Absorptionstestverfahren gemessene Diffusionsfläche der mittleren Schicht größer als oder gleich 1500 mm² ist.

5. Absorbierender Artikel (1) nach Anspruch 1, wobei der Absorber (4) einen Absorberkörper (20) umfasst, der eine Mischung aus Zellstoff und einem superabsorbierenden Polymer (12) ist, und der Vliesstoff (21) auf der Nicht-Hautseite des Absorberkörpers (20) angeordnet ist.

## Revendications

1. Article absorbant (1), comprenant :
au moins un absorbeur (4), sachant que
l'absorbeur (4) inclut une étoffe non tissée (21) disposée d'un côté non peau et présentant une diffusivité de fluide, l'étoffe non tissée (21) étant formée par empilement d'une couche filée-liée, d'une couche fusionnée-soufflée, et d'une couche filée-liée dans cet ordre et incluant un agent hydrophile ;
l'absorbeur (4) est une feuille polymère qui inclut une feuille de couche supérieure (10) disposée d'un côté peau, une feuille de couche inférieure (11) disposée du côté non peau, et un polymère superabsorbant (12) disposé entre la feuille de couche supérieure (10) et la feuille de couche inférieure (11) ;
la feuille de couche inférieure (11) est l'étoffe non tissée (21) ;
la feuille de couche supérieure (10) est composée d'une étoffe non tissée hydrophobe incluant des fibres chimiques ; et
l'étoffe non tissée (21) est configurée de telle sorte qu'une aire de diffusion de l'étoffe non tissée (21) soit supérieure ou égale à 1500 mm², l'aire de diffusion étant mesurée par un procédé de test d'absorption incluant :
(1) la préparation d'un échantillon d'une taille de 100 mm x 100 mm,
(2) le placement d'une pointe d'une burette à une hauteur de 10 mm au-dessus d'une surface de l'échantillon, et le versement d'une gouttelette d'eau ayant subi un échange d'ions depuis la burette, et
(3) après 3 minutes à compter d'un temps où la gouttelette atteint la surface de l'échantillon, la mesure, comme étant l'aire de diffusion, d'une aire d'une région de la surface de l'échantillon où une réflexion provenant de l'eau est nettement visible.

2. L'article absorbant (1) tel que revendiqué dans la revendication 1, sachant qu'un taux d'absorption de l'absorbeur (4) à un deuxième temps est inférieur ou égal à 42 secondes, le taux d'absorption étant mesuré par un procédé de test d'absorption incluant :
(1) après trois minutes à compter d'un temps où 1 cc de sang menstruel artificiel est injecté dans une surface, l'injection de 1 cc du sang menstruel artificiel dans une même position sur la surface ; et
(2) la mesure, comme étant le taux d'absorption, d'un temps entre une deuxième injection du sang menstruel artificiel et l'achèvement de l'absorption de tout le sang menstruel artificiel.

3. L'article absorbant (1) tel que revendiqué dans la revendication 1, sachant que la feuille de couche supérieure (10) est composée d'une étoffe non tissée incluant des fibres synthétiques thermoplastiques chimiques et formée par un procédé à passage d'air.

4. L'article absorbant (1) tel que revendiqué dans la revendication 1, sachant que
la feuille polymère inclut une feuille médiane disposée entre la feuille de couche supérieure (10) et la feuille de couche inférieure (11) ; et
le polymère superabsorbant (12) est disposé dans au moins un des espaces formés entre des feuilles adjacentes parmi la feuille de couche supérieure (10), la feuille de couche inférieure (11), et la feuille médiane ; et en plus de la feuille de couche inférieure (11), la feuille médiane est composée d'une étoffe non tissée et une aire de diffusion de la feuille médiane mesurée par le procédé de test d'absorption est supérieure ou égale à 1500 mm².

5. L'article absorbant (1) tel que revendiqué dans la revendication 1, sachant que l'absorbeur (4) inclut un corps d'absorbeur (20) qui est un mélange de pulpe et d'un polymère superabsorbant (12), et l'étoffe non tissée (21) est disposée du côté non peau du corps d'absorbeur (20).
